# EUROPEAN PATENT SPECIFICATION

(11) **EP 3 559 273 B1**
(45) Date of publication and mention of the grant of the patent: **13.07.2022**
(21) Application number: 17835850.3
(22) Date of filing: 22.12.2017
(51) Int. Cl.: C12Q 1/6816, C12Q 1/689

(54) **COBRA PROBES TO DETECT A MARKER FOR EPIDEMIC RIBOTYPES OF CLOSTRIDIUM DIFFICILE**
COBRA-SONDEN ZUM NACHWEIS EINES MARKERS FÜR EPIDEMISCHE RIBOTYPEN VON CLOSTRIDIUM DIFFICILE
SONDES COBRA POUR DÉTECTER UN MARQUEUR POUR DES RIBOTYPES ÉPIDÉMIQUES DE CLOSTRIDIUM DIFFICILE

(30) Priority: 22.12.2016 US 201662438331 P
(43) Date of publication of application: 30.10.2019
(73) Proprietor: Roche Diagnostics GmbH, 68305 Mannheim (DE); F. Hoffmann-La Roche AG, 4070 Basel (CH)
(72) Inventor: MANGIPUDI, Kalyani, Pleasanton, California 94588 (US); JOHNSON, Jenny A., Pleasanton, California 94588 (US); WILL, Stephen G., Pleasanton, California 94588 (US)
(74) Representative: Schwarz, Ralf
(86) International application number: PCT/EP2017/084323
(87) International publication number: WO 2018/115411

(56) References cited:
- WO-A1-2012/087135
- CN-A- 104 928 376
- US-A1- 2016 348 158

## Description

### FIELD OF THE INVENTION

The present invention relates to the field of microbial diagnostic, and more particularly, to the detection of a marker for epidemic ribotypes of *Clostridium difficile.*

### BACKGROUND OF THE INVENTION

*Clostridium difficile* (*"C. difficile"* or *"C. diff*") is an anaerobic, Gram-positive, spore-forming bacterium that can cause severe diarrhea and other intestinal disease. *C. difficile* spores are frequently found in hospitals, and although spores cannot cause infection directly, they can transform into the active infectious form when ingested. *C. difficile* infections include a wide range of clinical syndromes from simple diarrhea to pseudomembranous colitis associated with significant morbidity and mortality. Antibiotic-associated colitis is an infection of the colon caused by *C. difficile* that occurs when competing bacteria in the gut flora have been wiped out by antibiotics. It is the most common infection acquired by patients while they are in the hospital.

The majority of infections with *C. difficile* occur among persons aged 65 years or older and among patients in health-care facilities, such as hospitals and nursing homes. From 1996 to 2009, *C. difficile* rates for hospitalized persons aged 65 years or older increased 200%, with increases of 175% for those aged 65-74 years, 198% for those aged 75-84 years, and 201% for those aged 85 years or older. *C. difficile* rates among patients aged 85 years or older were notably higher than those for the other age groups (National Hospital Discharge Survey, Annual Files, 1996-2009).

The prevalence of hospital- and community-acquired *Clostridium difficile* infection (CDI) has recently increased, particularly in the Northern Hemisphere, with spread of the hypervirulent 027/NAPBI (Ribotype 027) strain. In pathogenic *C. difficile,* toxins A and B are encoded by *tcdA* and *tcdB,* respectively, and their expression is regulated by *tcdR* (positive regulator) and *tcdC* (negative regulator). It has been hypothesized that hypervirulence in the Ribotype 027 strain is due to increased toxin A/B production as a result of a single-base deletion at nucleotide position 117 in *tcdC* (tcdC Δ117) and the production of binary toxin CDT, encoded by *cdtA* and *cdtB* (MacCannell et al., Journal of Clinical Microbiology, 44:6, 2147-2152, 2006). There is also a characteristic 18-bp deletion in *tcdC* (nucleotide positions 330 to 347), which alone does not affect function (O'Connor et al., Gastroenterology, 136:1913-1924, 2009). Gene variations and mutations can be used to distinguish pathogens and in some cases are helpful in predicting prognosis and course of treatment. Therefore, there is a still a significant clinical need to develop molecular testing to detect variations and mutations in hypervirulent strains of C. *difficile* that is both sensitive and specific and also less susceptible to false positive or false negative results.

WO 2012/087135 A1 relates to the detection of C. difficile, ribotype 027 strain.

CN 104 928 376 A relates to composition, kit and method for detecting high-virulence bacterial strains and/or toxin type of clostridium difficile, and in particular of the ribotype 027 strain.

### SUMMARY OF THE INVENTION

The invention is set out in the appended set of claims.

The present invention relates to methods for the detection of the presence of the single-base deletion at nucleotide position 117 in the *tcdC* gene (tcdC Δ117) *of C. difficile* in a biological or nonbiological sample that is suspected to contain *C. difficile.* Herein, the present invention involves the use of "Cobra" probes, which have been disclosed in U.S. Pat. No. 8,409,802 and U.S. Patent No. 9,068,225. Briefly, Cobra probes are labeled with a detectable signal and have two domains separated by a linker section. The first domain is a 5' reporter sequence that is designed to be complementary to the region of a target nucleic acid that contains the gene variation or mutation (e.g. the single-base deletion in the *tcdC* gene). The second domain is a 3' anchor sequence that is designed to be fully complementary to the target nucleic acid and is tolerant of sequence mismatches or is designed to form a duplex with a region having low variability within the complementary region of the target nucleic acid. The reporter domain and the anchor domain of a Cobra probe are designed to bind to regions in the target nucleic acid that are adjacent to each other with only few intervening nucleotides when both reporter and anchor sequences are hybridized to the target nucleic acid. The linker section separates the reporter domain from the anchor domain and is comprised of a non-nucleoside linker that functions to keep the spacing between the reporter domain and the anchor domain.

Included are methods of detection of tcdC Δ117 comprising performing at least one cycling step, which may include an amplifying step and a hybridizing step. Furthermore, disclosed are primers, probes, and kits that are designed for the detection of tcdC Δ117.

In one aspect, provided are methods of detecting the presence or absence of tcdC Δ117 in a biological sample from an individual. Such methods generally include performing at least one cycling step, which includes an amplifying step and a dye-binding step or a hybridizing step using a fluorescently-labeled Cobra probe. Herein, the amplifying step includes contacting the sample with a pair of *tcdC* primers to produce a *tcdC* amplification product if a *tcdC* nucleic acid molecule is present in the sample and the detection step includes contacting the *tcdC* amplification product with a fluorescently-labeled Cobra probe that can specifically recognize presence or absence of tcdC Δ117 by hybridization.

More specifically, provided is a method for detecting a single-base deletion at nucleotide position 117 in the *tcdC* gene of Ribotype 027 strains *of C.* difficile (*tcdC* Δ117) in a sample, the method comprising performing an amplifying step comprising contacting the sample with a set of *tcdC* primers to produce an amplification product if *tcdC* nucleic acid of *C. difficile* is present in the sample; performing a hybridizing step comprising contacting the amplification product with a detectable Cobra probe designed to hybridize to tcdC Δ117 (e.g., a detectable tcdC Δ117 probe); and detecting the presence or absence of the amplified product (e.g., by detecting the presence or absence of a signal from said detectable tcdC Δ117 probe), wherein the presence of the amplified product (e.g., the presence of the signal) is indicative of the presence of tcdC Δ117 in the sample and wherein the absence of the amplified product (e.g., the absence of the signal) is indicative of the absence of tcdC Δ117 in the sample. Herein, the detectable tcdC Δ117 probe is a Cobra probe that comprises a reporter domain having a contiguous sequence that is fully complementary to the single-base deletion at nucleotide position 117 and wherein said reporter domain is separated from an anchor domain by a non-nucleoside linker comprising one or more hexaethylene glycol (HEG) and wherein said Cobra probe comprises a sequence selected from the group consisting of SEQ ID NOs: 1 and 2, or a complement thereof. In some embodiments, the hybridizing step comprises contacting the amplification product with said Cobra probe that is labeled with a donor fluorescent moiety and a corresponding acceptor fluorescent moiety; and the detecting step comprises detecting the presence or absence of fluorescence resonance energy transfer (FRET) between the donor fluorescent moiety and the acceptor fluorescent moiety of the Cobra probe, wherein the presence or absence of fluorescence is indicative of the presence or absence of tcdC Δ117 in the sample. In some embodiments, said amplification employs a polymerase enzyme having 5' to 3' exonuclease activity. In some embodiments, the tcdC Δ117 probe comprises a nucleic acid sequence that permits secondary structure formation, wherein the secondary structure formation results in spatial proximity between the donor fluorescent moiety and the acceptor fluorescent moiety. In some embodiments, said second fluorescent moiety is a quencher. In some embodiments, the amplifying step and the hybridizing step are repeated at least once. In some embodiments, the amplifying step and the hybridizing step are repeated at least about 20 times, but may be repeated as many as at least 40, at least 60, or at least 100 times. In some embodiments, the method comprises performing multiple cycling steps, wherein each cycling step comprises an amplifying step comprising contacting the sample with a set of *tcdC* primers to produce an amplification product if a *tcdC* nucleic acid is present in the sample and a hybridizing step comprising contacting the amplification product with a detectable tcdC Δ117 probe. Herein, the cycling steps may be repeated at least about 20 times, but may be repeated as many as at least 40, as at least 60, or even at least 100 times.

In a further aspect, provided is a kit for detecting a nucleic acid of tcdC Δ117. The kit includes a detectably labeled oligonucleotide (the Cobra probe) comprising or consisting of a sequence selected from the group consisting of SEQ ID NOs: 1 and 2, or a complement thereof. More specifically, the present invention provides a kit for detecting a single-base deletion at nucleotide position 117 in the tcdC gene of Ribotype 027 strains of C. difficile (tcdC Δ117) comprising one pair of oligonucleotide primers, each oligonucleotide primer capable of hybridizing to opposite strands of a subsequence of the *tcdC* gene containing nucleotide position 117 and a detectably labeled oligonucleotide probe comprising or consisting of a sequence selected from the group consisting of SEQ ID NOs:1 and 2, or a complement thereof. In some embodiments, the detectably labeled oligonucleotide comprises a donor fluorescent moiety and a corresponding acceptor fluorescent moiety. In some embodiments, the acceptor fluorescent moiety is a quencher. In some embodiments, the kit further comprises nucleoside triphosphates, a nucleic acid polymerase, and buffers necessary for the function of the nucleic acid polymerase. In some embodiments, the kit can also include a package insert and instructions for using the primers, probes, and fluorophoric moieties to detect the presence or absence of tcdC Δ117 in a sample.

In another aspect, provided is an oligonucleotide comprising or consisting of a sequence of nucleotides selected from SEQ ID NOs: 1 or 2 or a complement thereof. In some embodiments, the oligonucleotide has 100 or fewer nucleotides. Also disclosed is an oligonucleotide that includes a nucleic acid having at least 70% sequence identity (e.g., at least 75%, 80%, 85%, 90% or 95%, etc.) to one of SEQ ID NOs: 1 or 2 or a complement thereof, which oligonucleotide has 100 or fewer nucleotides. Generally, these oligonucleotides are probe nucleic acids, or the like in these embodiments. In certain of these embodiments, the oligonucleotides have 50 or fewer nucleotides (e.g., 45 or fewer nucleotides, 40 or fewer nucleotides, etc.). In some embodiments, the oligonucleotides comprise at least one modified nucleotide, e.g., to alter nucleic acid hybridization stability relative to unmodified nucleotides. In some embodiments, the oligonucleotide comprises at least one conservatively modified variation. In some embodiments, the oligonucleotide comprises one or more detectable label. In some embodiments, the oligonucleotide comprises at least one labeling moiety and/or at least one quencher moiety.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

The details of one or more embodiments of the invention are set forth in the accompanying drawings and the description below. Other features, objects, and advantages of the invention will be apparent from the drawings and detailed description, and from the claims.

### BRIEF DESCRIPTION OF THE FIGURES

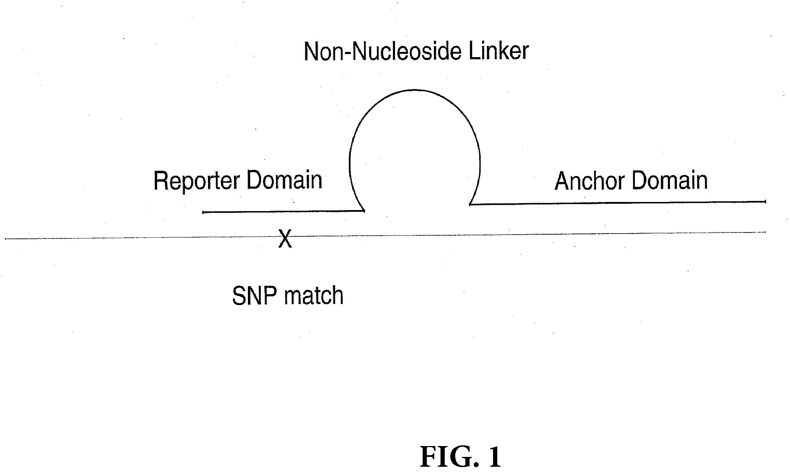

FIG. 1 is a graphical representation of a Cobra probe that shows the Reporter Domain and the Anchor Domain separated by the Non-Nucleoside Linker.

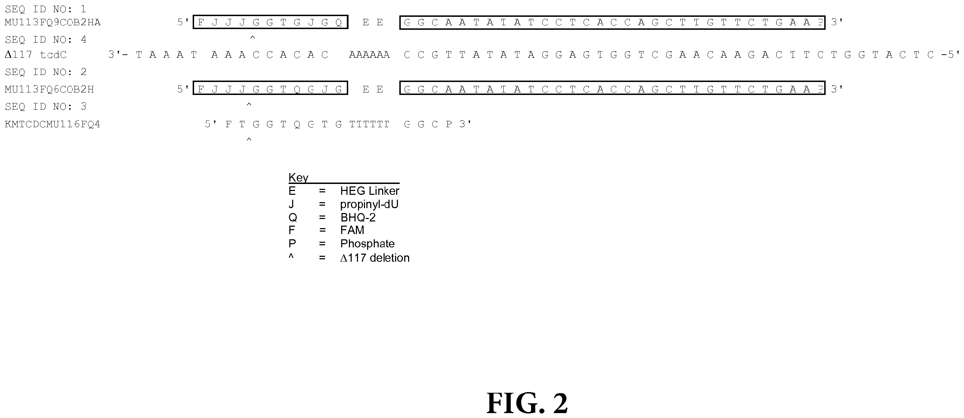

FIG. 2 shows the Cobra probes designed to detect tcdC Δ117 as described in Example 1.

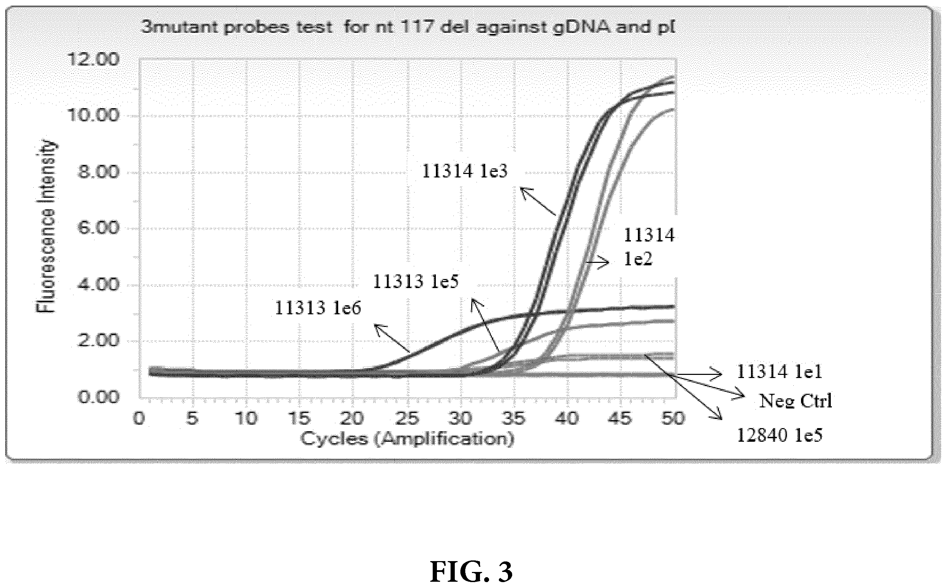

FIG. 3 shows the PCR growth curves generated by different starting concentrations of genomic *C. difficile* DNA from wild-type strain (12840), strain with an SNP at nucleotide position 120, (11313) or strain with Δ117 deletion (11314), measured as FAM signals in the presence of the regular Δ117probe (Δ117-sense FQ4) of SEQ ID NO:3.

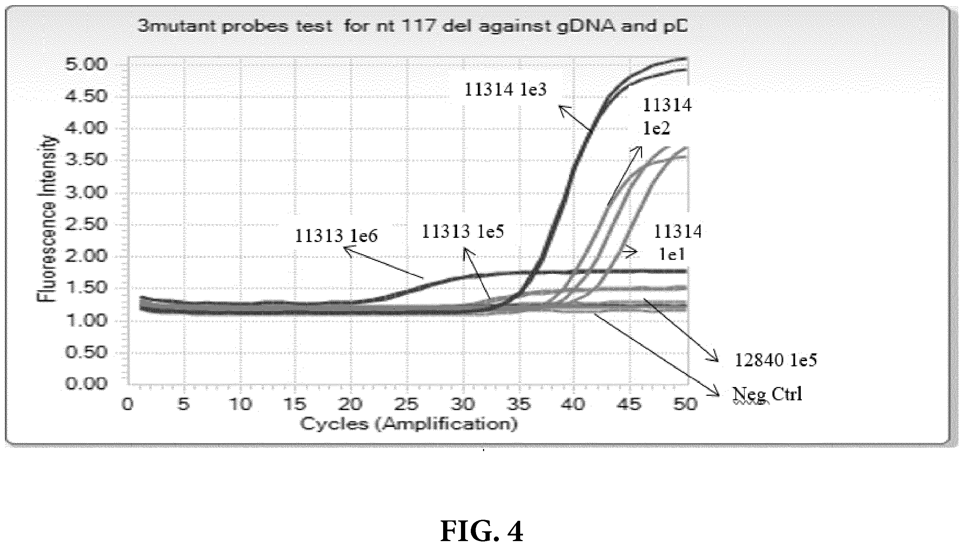

FIG. 4 shows the PCR growth curves generated by different starting concentrations of genomic *C. difficile* DNA from wild-type strain (12840), strain with an SNP at nucleotide position 120 (11313), or strain with Δ117 deletion (11314), measured as FAM signals in the presence of the Cobra Δ117probe (Δ117-Cobra FQ6) of SEQ ID NO:2.

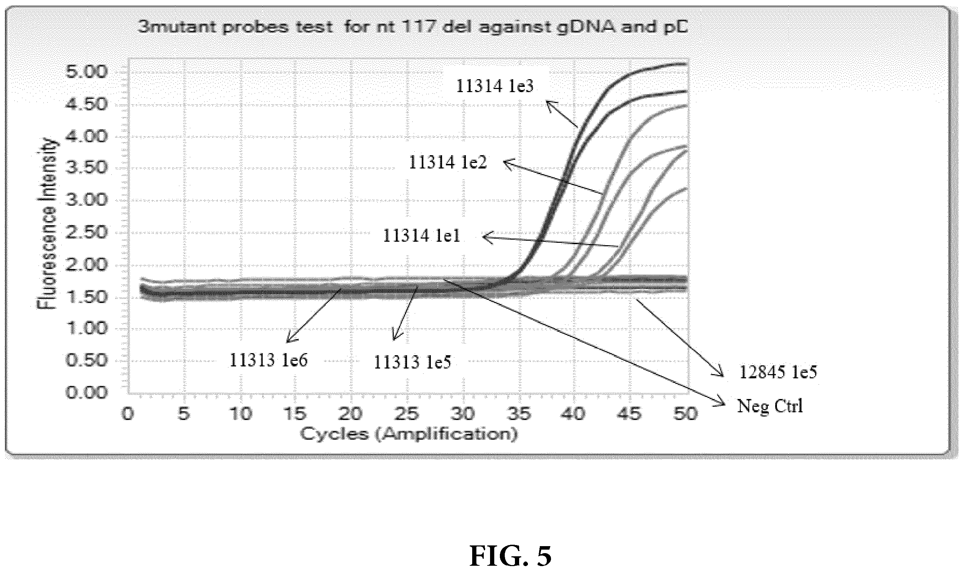

FIG. 5 shows the PCR growth curves generated by different starting concentrations of genomic *C. difficile* DNA from wild-type strain (12840), strain with an SNP at nucleotide position 120 (11313), or strain with Δ117 deletion (11314), measured as FAM signals in the presence of the Cobra Δ117probe (Δ117-Cobra FQ9) of SEQ ID NO:1.

### DETAILED DESCRIPTION OF THE INVENTION

A real-time assay for detecting the single-base deletion at nucleotide position 117 in the *tcdC* gene of Ribotype 027 strains of *C. difficile* (tcdC Δ117) in a sample is described herein. Cobra probes for detecting tcdC Δ117 are provided, as are articles of manufacture or kits containing such probes. The increased sensitivity of real-time PCR for detection of tcdC Δ117compared to other methods, as well as the improved features of real-time PCR including sample containment and real-time detection of the amplified product, make feasible the implementation of this technology for diagnosis of tcdC Δ117 in the clinical laboratory.

The methods may include performing at least one cycling step that includes amplifying a portion of a *tcdC* nucleic acid molecule from a sample using a pair of *tcdC* primers. *"tcdC* primers" as used herein refers to oligonucleotide primers that specifically anneal to nucleic acid sequences encoding *tcdC* and initiate synthesis therefrom under appropriate conditions. Each of the *tcdC* primers anneals to a target within or adjacent to a *tcdC* nucleic acid molecule such that at least a portion of each amplification product contains nucleic acid sequence corresponding to *tcdC.* The *tcdC* amplification product is produced provided that *tcdC* nucleic acid is present in the sample, thus the presence of the *tcdC* amplification product is indicative of the presence of *C. difficile* in the sample. The amplification product should contain the nucleic acid sequences that are complementary to one or more detectable *tcdC* probes. Each cycling step includes an amplification step, a hybridization step, and a detection step, in which the sample is contacted with the one or more detectable *tcdC* probes for detection of the presence or absence of C. *difficile* in the sample.

As used herein, the term "amplifying" refers to the process of synthesizing nucleic acid molecules that are complementary to one or both strands of a template nucleic acid molecule (e.g., *tcdC* nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (e.g., Platinum^{®} Taq) and an appropriate buffer and/or co-factors for optimal activity of the polymerase enzyme (e.g., MgCl₂ and/or KCl).

"Conservatively modified variations" or, simply, "conservative variations" of a particular nucleic acid sequence refers to those nucleic acids, which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid.

The term "primer" is used herein as known to those skilled in the art and refers to oligomeric compounds, primarily to oligonucleotides but also to modified oligonucleotides that are able to "prime" DNA synthesis by a template-dependent DNA polymerase, i.e., the 3'-end of the, e.g., oligonucleotide provides a free 3'-OH group whereto further "nucleotides" may be attached by a template-dependent DNA polymerase establishing 3' to 5' phosphodiester linkage whereby deoxynucleoside triphosphates are used and whereby pyrophosphate is released. Therefore, there is - except possibly for the intended function - no fundamental difference between a "primer", an "oligonucleotide", or a "probe" according to the invention.

The term "hybridizing" refers to the annealing of one or more probes to an amplification product. Hybridization conditions typically include a temperature that is below the melting temperature of the probes but that avoids non-specific hybridization of the probes.

The term "5' to 3' exonuclease activity" refers to an activity of a nucleic acid polymerase, typically associated with the nucleic acid strand synthesis, whereby nucleotides are removed from the 5' end of nucleic acid strand.

The term "thermostable polymerase" refers to a polymerase enzyme that is heat stable, i.e., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus fiavus, T. ruber, T. thermophilus*, *T. aquaticus, T. lacteus*, *T. rubens, Bacillus stearothermophilus,* and *Methanothermus fervidus.* Nonetheless, polymerases that are not thermostable also can be employed in PCR assays provided the enzyme is replenished.

The term "complement thereof' refers to nucleic acid that is both the same length as, and exactly complementary to, a given nucleic acid.

The term "extension" or "elongation" when used with respect to nucleic acids refers to when additional nucleotides (or other analogous molecules) are incorporated into the nucleic acids. For example, a nucleic acid is optionally extended by a nucleotide incorporating biocatalyst, such as a polymerase that typically adds nucleotides at the 3' terminal end of a nucleic acid. The terms "identical" or percent "identity" in the context of two or more nucleic acid sequences, refer to two or more sequences or subsequences that are the same or have a specified percentage of nucleotides that are the same, when compared and aligned for maximum correspondence, e.g., as measured using one of the sequence comparison algorithms available to persons of skill or by visual inspection. Exemplary algorithms that are suitable for determining percent sequence identity and sequence similarity are the BLAST programs, which are described in, e.g., Altschul et al. (1990) "Basic local alignment search tool" J. Mol. Biol. 215:403-410, Gish et al. (1993) "Identification of protein coding regions by database similarity search" Nature Genet. 3:266-272, Madden et al. (1996) "Applications of network BLAST server" Meth. Enzymol. 266:131-141, Altschul et al. (1997) "Gapped BLAST and PSI-BLAST: a new generation of protein database search programs" Nucleic Acids Res. 25:3389-3402, and Zhang et al. (1997) "PowerBLAST: A new network BLAST application for interactive or automated sequence analysis and annotation" Genome Res. 7:649-656.

A "modified nucleotide" in the context of an oligonucleotide refers to an alteration in which at least one nucleotide of the oligonucleotide sequence is replaced by a different nucleotide that provides a desired property to the oligonucleotide. Exemplary modified nucleotides that can be substituted in the oligonucleotides described herein include, e.g., a C5-methyl-dC, a C5-ethyl-dC, a C5-methyl-dU, a C5-ethyl-dU, a 2,6-diaminopurine, a C5-propynyl-dC, a C5-propynyl-dU, a C7-propynyl-dA, a C7-propynyl-dG, a C5-propargylamino-dC, a C5-propargylamino-dU, a C7-propargylamino-dA, a C7-propargylamino-dG, a 7-deaza-2-deoxyxanthosine, a pyrazolopyrimidine analog, a pseudo-dU, a nitro pyrrole, a nitro indole, 2'-0-methyl Ribo-U, 2'-0-methyl Ribo-C, an N4-ethyl-dC, an N6-methyl-dA, and the like. Many other modified nucleotides that can be substituted in the oligonucleotides of the invention are referred to herein or are otherwise known in the art. In certain embodiments, modified nucleotide substitutions modify melting temperatures (Tm) of the oligonucleotides relative to the melting temperatures of corresponding unmodified oligonucleotides. To further illustrate, certain modified nucleotide substitutions can reduce non-specific nucleic acid amplification (e.g., minimize primer dimer formation or the like), increase the yield of an intended target amplicon, and/or the like in some embodiments of the invention. Examples of these types of nucleic acid modifications are described in, e.g., U.S. Pat. No. 6,001,611.

### C. difficile Nucleic Acids and Oligonucleotides

The invention provides methods to detect epidemic ribotypes of *C. difficile* (e.g. Ribotype 027) by amplifying, for example, a portion of the *tcdC* gene. Nucleic acid sequences of *tcdC* gene from *C. difficile* are available (see, for example, GenBank Accession No. AM180355. Specifically, primers and probes to amplify and detect *tcdC* nucleic acid molecules are provided by the present invention. Furthermore, Cobra probes to specifically detect the single-base deletion at nucleotide position 117 in the *tcdC* gene, tcdC Δ117, are provided by the present invention.

More specifically, the oligonucleotides of the present invention each include a nucleic acid with a sequence selected from SEQ ID NOs: 1 and 2, a substantially identical variant thereof in which the variant has at least, e.g., 80%, 90%, or 95% sequence identity to one of SEQ ID NOs: 1 and 2, or a complement of SEQ ID NOs: 1 and 2 and the variant.

In one embodiment of the invention, a particular set of *tcdC* primers and a Cobra probe specific for tcdC Δ117 is used in order to provide for detection of tcdC Δ117 in a biological sample suspected of containing C. *difficile.* The set of primers and probe may comprise at least one primer and probe specific for *tcdC.* A functionally active variant of the Cobra probes of SEQ ID NOs: 1 and 2 may be identified by using the probes in the method of the invention. A functionally active variant of a Cobra probe of SEQ ID NOs: 1 and 2 pertains to a probe which provides a similar or higher specificity and sensitivity in the method or kit of the invention as compared to the respective sequence of SEQ ID NOs: 1 and 2.

The variant may, e.g., vary from the sequence of SEQ ID NOs: 1, and 2by one or more nucleotide additions, deletions or substitutions such as one or more nucleotide additions, deletions or substitutions at the 5' end and/or the 3' end of the respective sequence of SEQ ID NOs: 1 and 2. As detailed above, a probe may be chemically modified, i.e., a probe may comprise a modified nucleotide or a non-nucleotide compound. A probe is then a modified oligonucleotide. "Modified nucleotides" (or "nucleotide analogs") differ from a natural "nucleotide" by some modification but still consist of a base or base-like compound, a pentofuranosyl sugar or a pentofuranosyl sugar-like compound, a phosphate portion or phosphate-like portion, or combinations thereof. For example, a "label" may be attached to the base portion of a "nucleotide" whereby a "modified nucleotide" is obtained. A natural base in a "nucleotide" may also be replaced by, e.g., a 7-desazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

Oligonucleotides including modified oligonucleotides and oligonucleotide analogs that amplify a nucleic acid molecule encoding *tcdC,* e.g., nucleic acids encoding alternative portions of *tcdC,* can be designed using, for example, a computer program such as OLIGO (Molecular Biology Insights Inc., Cascade, Colo.). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 8 to 50 nucleotides in length (e.g., 8, 10, 12, 14, 16, 18, 20, 22, 24, 26, 28, 30, 32, 34, 36, 38, 40, 42, 44, 46, 48, or 50 nucleotides in length).

In addition to a set of primers, the methods of the invention may use one or more probes in order to detect the presence or absence of tcdC Δ117. The term "probe" refers to synthetically or biologically produced nucleic acids (DNA or RNA), which by design or selection, contain specific nucleotide sequences that allow them to hybridize under defined predetermined stringencies specifically (i.e., preferentially) to "target nucleic acids", in the present case to a *tcdC* (target) nucleic acid. A "probe" can be referred to as a "detection probe" meaning that it detects the target nucleic acid.

According to the invention, the *tcdC* probe can be labeled with at least one fluorescent label. In one embodiment, the *tcdC* probe can be labeled with a donor fluorescent moiety, e.g., a fluorescent dye, and a corresponding acceptor fluorescent moiety, e.g., a quencher.

In one embodiment of the present invention, at least one probe comprises or consists of a fluorescent moiety and a nucleic acid sequences selected from the group consisting of SEQ ID NOs: 1 and 2 (shown without the label).

Designing oligonucleotides to be used as hybridization probes can be performed in a manner similar to the design of primers. Embodiments of the present invention may use a single probe or a pair of probes for detection of the amplification product. Depending on the embodiment, the probe(s) use may comprise at least one label and/or at least one quencher moiety. As with the primers, the probes usually have similar melting temperatures, and the length of each probe must be sufficient for sequence-specific hybridization to occur but not so long that fidelity is reduced during synthesis. Oligonucleotide probes are generally 30 to 50 (nucleotides in length. Constructs of the present invention include vectors containing a *tcdC* nucleic acid molecule. Constructs of the invention can be used, for example, as control template nucleic acid molecules. Vectors suitable for use in the present invention are commercially available and/or produced by recombinant nucleic acid technology methods routine in the art. *TcdC* nucleic acid molecules can be obtained, for example, by chemical synthesis, direct cloning from *C. difficile* or by PCR amplification.

Constructs suitable for use in the methods of the invention typically include, in addition to *tcdC* nucleic acid molecules sequences encoding a selectable marker (e.g., an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery. Constructs of the invention containing *tcdC* nucleic acid molecules can be propagated in a host cell. As used herein, the term host cell is meant to include prokaryotes and eukaryotes such as yeast, plant and animal cells. Prokaryotic hosts may include *E. coli, Salmonella typhimurium, Serratia marcescens,* and *Bacillus subtilis.* Eukaryotic hosts include yeasts such as *S. cerevisiae, S. pombe, Pichia pastoris,* mammalian cells such as COS cells or Chinese hamster ovary (CHO) cells, insect cells, and plant cells such as *Arabidopsis thaliana* and *Nicotiana tabacum.* A construct of the invention can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, e.g., U.S. Pat. Nos. 5,580,859 and 5,589,466).

### Cobra Probes

Cobra Probes that are used in the present invention are described in U.S. Patent No. 8,409,802 and U.S. Patent No. 9,068,225. A Cobra probe refers to a two-domain probe having a flexible linker that allows for specific detection of the presence or absence of a target nucleic acid sequence. The first domain serves as an "anchor" that drives the hybridization of the probe onto the target nucleic acid. This domain hybridizes to the target nucleic acid stably, and is tolerant of potential mismatches, or is designed to form a duplex with a region having low variability within the complementary region ("anchor-binding" region) of the target nucleic acid. The second domain serves as a "reporter" that is designed to detect the sequence of interest. The "reporter" is highly specific for the complementary region of the target nucleic acid ("reporter-binding" region), and is capable of detecting the presence or absence of the target sequence as well as differentiation of match/mismatch with the reporter binding region. The reporter domain is generally linked to a label such that one can distinguish hybridization and lack of hybridization of the reporter domain, thus detecting the presence or absence of the target sequence, or detect alterations in melting temperature, thus detecting mismatches in the probe region. The two-domain probe of this design allows for highly specific in detecting a target sequence of interest.

A Cobra probe is designed to be detectably labeled to detect the presence or absence of a target nucleic acid, i.e. such that there is a change in signal from the label when the reporter domain hybridizes to the target compared to when the reporter is not hybridized to the target. By detecting and measuring the change in the detectable signal, the presence or absence of the target nucleic acid sequence can be qualitatively or quantitatively determined. By detecting alterations in melting temperature, the presence or absence of mutations or alterations of the target can be determined.

The anchor domain of the Cobra probe is designed to hybridize to an "anchor binding" sequence of the target nucleic acid under the conditions of the assay. In contrast, the reporter domain may or may not hybridize to its target "binding region" depending on whether the precise target sequence is present. The reporter domain can be designed, for example, to be complementary or at least substantially complementary to a "reporter binding" sequence of a target nucleic acid. Depending on the reaction conditions used, one can distinguish hybridization of the reporter domain to the target versus hybridization to a target variant sequence.

The Cobra probe can be designed to act as a primer that permits chain extension or elongation using, e.g., a nucleotide incorporating biocatalyst, such as a polymerase under appropriate reaction conditions. Alternatively, the probe can also be rendered non-extendible such that additional nucleotides cannot be added to the probe. The probe can be rendered non-extendible, for example, by modifying the 3' end of the probe such that the hydroxyl group is no longer capable of participating in elongation. The hydroxyl group of a 3' natural-occurring nucleotide simply can be modified with a variety of functional groups. For example, the 3' end of the probe can be rendered non-extendible by incorporating a nucleotide analog that lacks a 3' hydroxyl group or cannot function as a substrate of a polymerase for extension. Optionally, the probe can be rendered non-extendible by incorporating a label on its 3' end.

In some embodiments, Cobra probes can be rendered non-extendible by incorporating a 2'-terminator nucleotide, a nucleotide analog having a blocking group at the 2'-position of the sugar moiety of the nucleotide. A "blocking group" refers to a chemical group or moiety that typically prevents the extension of a nucleic acid (i.e., a 2'-terminator nucleotide is typically non-extendible by one or more nucleotide incorporating biocatalysts). That is, once a 2'-terminator nucleotide is incorporated into a nucleic acid (e.g., at a 3'-terminus end of the nucleic acid), the blocking group prevents further extension of a nucleic acid by at least one nucleotide incorporating biocatalyst. Exemplary nucleotide incorporating biocatalysts include DNA polymerases, including but not limited to, e.g., a G46E E678G CS5 DNA polymerase, a G46E L329A E678G CS5 DNA polymerase, a G46E L329A D640G S671F CS5 DNA polymerase, a G46E L329A D640G S671F E678G CS5 DNA polymerase, a G46E E678G CS6 DNA polymerase, a ΔZO5R polymerase, a E615G Taq DNA polymerase, a Thermus flavus polymerase, a TMA-25 polymerase, a TMA-30 polymerase, a Tth DNA polymerase, a Thermus specie SPS-17 polymerase, a E615G Taq polymerase, a Thermus Z05R polymerase, a T7 DNA polymerase, a Kornberg DNA polymerase I, a Klenow DNA polymerase, a Taq DNA polymerase, a Micrococcal DNA polymerase, an alpha DNA polymerase, a reverse transcriptase, an AMV reverse transcriptase, an M-MuLV reverse transcriptase, a DNA polymerase, an RNA polymerase, a E. coli RNA polymerase, an SP6 RNA polymerase, a T3 RNA polymerase, a T4 DNA polymerase, a T7 RNA polymerase, an RNA polymerase II, a terminal transferase, a polynucleotide phosphorylase, a ribonucleotide incorporating DNA polymerase, and the like.

An exemplary blocking group is a phosphate group. Exemplary 2'-terminator nucleotides include 2'-monophosphate-3'-hydroxyl-5'-triphosphate nucleosides and 2'-monophosphate-3'-hydroxyl-5'-diphosphate nucleosides.

In a Cobra probe, the anchor nucleic acid domain and the reporter nucleic acid domain is linked by a non-nucleoside linker. Any standard technique can be used to attach the anchor nucleic acid domain or the reporter nucleic acid domain to the non-nucleoside linker. The anchor nucleic acid domain or the reporter nucleic acid domain can be linked to the non-nucleoside linker either directly or indirectly. In some embodiments, the anchor nucleic acid domain or the reporter nucleic acid domain is covalently attached to the non-nucleoside linker.

The anchor nucleic acid domain is designed to bind to a "anchor binding" sequence of a target nucleic acid in a biological sample under the conditions of the assay. Accordingly, the anchor nucleic acid domain can be designed to comprise a region that is complementary or substantially complementary to the "anchor binding" sequence of the target nucleic acid. In some embodiments of the invention, the anchor nucleic acid domain comprises, or consists of, a region that is complementary or substantially complementary to the "anchor binding" sequence within the target nucleic acid. The complementary regions can be of any length including but not limited to, at least 4, 6, 8, 10, 12, 14, 16, 20 or more nucleotides, e.g., 3-50, 5-20, 5-50, or 8-25 nucleotides long.

The anchor nucleic acid domain can comprise additional regions, for example, a region for attachment of detectable labels. In some embodiments, the anchor nucleic acid domain contains only one region that is complementary or substantially complementary to one "anchor binding" sequence of the target nucleic acid. In some embodiments, the anchor nucleic acid domain can contain two or more regions, each binds to a distinct "anchor binding" sequence within the target nucleic acid.

The anchor nucleic acid domain (or the first domain) is designed to drive the hybridization of the probe to the target nucleic acid. In some embodiments, the composition and length of the anchor nucleic acid domain is chosen to overcome (i.e., hybridize under conditions of the method in spite of) potential mismatches between the anchor nucleic acid domain and the "anchor binding" sequence due to the infrequent polymorphisms within the "anchor binding" sequence. The anchor nucleic acid domain can be of any length as long as the affinities between the anchor nucleic acid domain and the "anchor binding" sequence is sufficiently large such that they hybridize under the reaction conditions. In some embodiments, the length of the anchor nucleic acid domain is chosen so that there is sufficient specificity between the probe and the target nucleic acid such that they hybridize under the reaction conditions. In some embodiments, the hybridization between the anchor nucleic acid domain and the "anchor binding" sequence alone (i.e., whether or not the reporter domain hybridizes to the target) is sufficient to drive the hybridization of the probe onto the intended location of the target nucleic acid.

In some embodiments, the anchor nucleic acid domain is about five nucleotides to about 200 nucleotides in length. In some embodiments, the anchor nucleic acid domain is between 4 to 50 nucleotides in length. In some embodiments, the anchor nucleic acid domain is between 6 to 40 nucleotides in length. In some embodiments of the invention, the anchor nucleic acid domain is between 20 to 40 nucleotides in length. In some embodiments of the invention, the anchor nucleic acid domain is between 8 to 25 nucleotides in length. In some embodiments of the invention, the anchor nucleic acid domain is about 30 nucleotides in length. In other embodiments of the invention, the anchor nucleic acid domain is between 40 to 60 nucleotides in length. In some other embodiments of the invention, the anchor nucleic acid domain is more than 60 nucleotides in length.

The anchor nucleic acid domain can comprise natural nucleotides, non-natural nucleotides, or combinations thereof. Exemplary nucleic acids include, but are not limited to, conventional ribonucleic acid (RNA), deoxyribonucleic acid (DNA), and chemical analogs of these molecules such as a locked nucleotide analog ("LNA") and a peptide nucleic acid ("PNA"). Various nucleic acid analogs described herein can also be used for the anchor nucleic acid domain. In some embodiments, the non-natural nucleotide increases the melting temperature of the anchor domain compared to a corresponding natural nucleotide in the place of the non-natural nucleotide. Exemplary artificial bases that contribute to increased Tm are described in the art, including but not limited to, e.g., Lebedev et al., Geneteic Analysis - Biomolecular Engineering 13:15-21 (1996); Xodo, et al., Nucleic Acids Res. 19:5625-5631 (1991); Froehler, et al., Tetrahedron Lett. 33:5307-5310 (1992); Kutyavin, et al., Biochemistry 35:11170-11176 (1996); Nguyen, et al., Nucleic Acids Res. 25:30599-65 (1997). In some embodiments, the non-natural nucleotides are propynyl-dN. In some embodiments of the invention, thymine is replaced with propynyl dU, cytosine is replaced with propynyl dC.

The reporter nucleic acid domain is designed to detect the presence or absence of a target nucleic acid sequence in a biological sample. Accordingly, the reporter nucleic acid domain can be designed to comprise a region that is complementary or substantially complementary to a "reporter binding" sequence within the target nucleic acid. As discussed herein, the anchor binding domain of the target and the reporter binding domain of the target are on the same strand of target nucleic acid. In some embodiments, the reporter nucleic acid domain comprises, or consists of, a region that is complementary or substantially complementary to a "reporter binding" sequence within the target nucleic acid. In other embodiments, the reporter nucleic acid domain comprises additional regions, for example, a region for attachment of detectable labels. In some embodiments, the reporter nucleic acid domain contains only one region that is complementary or substantially complementary to one "reporter binding" sequence of the target nucleic acid. In some embodiments, the reporter nucleic acid domain can contain two or more regions, each of which is capable of hybridizing to a distinct "reporter binding" sequence of the target nucleic acid, for example, multiple SNP regions within the target nucleic acid. Because the reporter domain sequence is designed to be selective for a specific reporter domain-binding sequence, generally the region of the reporter domain is fully complementary to the reporter binding sequence. Alternatively, the reporter domain, in combination with the reaction conditions, can be designed so that the reporter domain hybridizes to a specific target or to the specific target and to slight (e.g., a single nucleotide mismatch or deletion) variants, but does not bind to more widely varying sequences (e.g., larger deletions or substitutions, rearrangements, more than single mismatches, etc.).

The reporter nucleic acid domain and detectable label can be designed to detect the presence or absence of any target nucleic acid of interest. The target nucleic acid can be from any source. For example, the target nucleic acid can be a viral or microbial nucleic acid. Alternatively, the target nucleic acid can also be, e.g., an animal, mammalian, human, fungal or plant nucleic acid. In some embodiments, the reporter nucleic acid domain of the present invention can be designed to detect the presence or absence of a single nucleotide polymorphism (SNP) or a mutation within the target nucleic acid. Accordingly, in some embodiments, the reporter nucleic acid domain comprises a binding region that is 100% complementary to one allele of the SNP or the mutation and the probe is used under conditions that allow for one to distinguish between binding to the one allele and other possible alleles of the SNP or the wild-type sequence.

The reporter nucleic acid domain thus can be designed to be highly specific to the "reporter binding" sequence. In some embodiments, even a single-base mismatch, a single-base insertion or a single-base insertion between the reporter nucleic acid domain and the "reporter binding" sequence can induce a signal change, either chemical or physical in nature, significant enough to facilitate the detection of the match (or the mismatch). The signal change can be a conformational change of the probe, i.e. from a random coil to a duplex formed between the reporter nucleic acid domain and the target nucleic acid. The signal change can be physically or chemically detectable. For example, the probe can be fluorescently labeled in a way that fluorescence signal can be induced or changed upon a match or mismatch. The label and the probe can be designed such that a difference in fluorescence is detected between hybridization and no hybridization between the reporter nucleic acid domain and the "reporter binding" sequence. In some embodiments, this difference does not involve a stem loop in either domain of the probe (e.g., in the absence of the target). The signal change can also be a change in the melting temperature of the hybridizing duplex formed between the reporter nucleic acid domain and the "reporter binding" sequence. Tm change can be measured, for example, between a complementary duplex and a mismatched duplex formed between the reporter nucleic acid domain and the "reporter binding" sequence.

In some embodiments, the report nucleic acid domain comprises, or consists of, a region that is complementary or substantially complementary to the "reporter binding" sequence within the target nucleic acid. The complementary regions can be of any length useful for the invention, including but not limited to, at least 4, 6, 8, 10, 12, 14, 16, 20 or more nucleotides, e.g., 3-50, 5-20, 5-50, or 8-25 nucleotides long.

The reporter nucleic acid domain can be of any suitable length. In some embodiments, to maximize the specificity for its complementary sequence, the reporter nucleic acid domain of the present invention is designed to be relatively short in length. In some embodiments, the length of the target-binding portion of the reporter nucleic acid domain is between 4 to 20 nucleotides. In some embodiments, the length of the reporter nucleic acid domain is between 6 to 12 nucleotides. In some embodiments, the length of the reporter nucleic acid domain is between 8 to 25 nucleotides. In some embodiments, the length of the reporter nucleic acid domain is between 6 to 40 nucleotides. In some embodiments, the reporter nucleic acid domain is at least 6, 7, 8, 9, 10, 11, or 12 nucleotides in length.

The Cobra probe takes advantage of the high specificity of the reporter nucleic acid domain for the "reporter binding" sequence. Accordingly, the reporter nucleic acid domain can comprise a non-natural nucleotide to increase the binding specificity of the reporter nucleic acid domain, and therefore increase the probe differentiation between a match and a mismatch. In some embodiments of the invention, the non-natural nucleotide increases the melting temperature of the reporter domain compared to a corresponding natural nucleotide in the place of the non-natural nucleotide. In some embodiments of the invention, the non-natural nucleotide increases the difference in Tm of a match and a mismatch. In some embodiments of the invention, the non-natural nucleotides are propynyl-dN. Exemplary artificial bases that contribute to increased Tm are described in the art, including but not limited to, e.g., Lebedev et al., Geneteic Analysis - Biomolecular Engineering 13:15-21 (1996); Xodo, et al., Nucleic Acids Res. 19:5625-5631 (1991); Froehler, et al., Tetrahedron Lett. 33:5307-5310 (1992); Kutyavin, et al., Biochemistry 35:11170-11176 (1996); Nguyen, et al., Nucleic Acids Res. 25:30599-65 (1997). For example, 2-Amino A increases Tm by about 3°C over A, 5-Methyl-C raises the Tm about 1.3°C over C, C-5 propynyl-C improves the Tm about 2.8°C over C and C-5 propynyl-U increases the Tm about 1.7°C over T. optionally, the reporter region will comprise one or more (e.g., 2, 3, 4, 5, 6, 7, 8, 9, or more) stabilizing bases, but one or no stabilizing (e.g., non-natural) bases in the portion of the reporter region to hybridize to a potentially variable sequence (e.g., a SNP or expected possible mutation).

The anchor nucleic acid domain and the reporter nucleic acid domain can adopt any conformation in the absence of a target nucleic acid. In some embodiments, neither the anchor nor the reporter domain forms a stem loop. In some embodiments, the anchor nucleic acid domain and the reporter nucleic acid domain are designed to have no particular secondary structure (e.g. a random coil). In some embodiments, some regions of these domains can hybridize to each other. For example, a region in the anchor nucleic acid domain may be complementary or partially complementary to a region in the reporter nucleic acid domain. In some embodiments, the anchor and reporter domains are less than 50% complementary. In some embodiments, the probe has a stem loop structure in the absence of a target nucleic acid, formed between complementary or partially complementary regions (e.g., regions of at least 4 contiguous nucleotides) in the anchor domain and the reporter domain. In other embodiments, the anchor domain can form a stem loop in the absence of a target nucleic acid. Alternatively, the reporter domain can form a stem loop in the absence of a target nucleic acid.

In some embodiments, neither the anchor nor the reporter domains comprise a stem loop. Thus, in some embodiments, the reporter domain does not include two sequences of at least three nucleotides that are fully complementary and wherein the two sequences have at least two nucleotides intervening; and/or the anchor domain does not include two sequences of at least three nucleotides that are fully complementary and wherein the two sequences have at least two nucleotides intervening.

In some embodiments, the anchor is at the 3' end of the probe and the reporter is at the 5' end of the probe. While Figure 1 illustrates an embodiment wherein the reporter is linked to the 5' end of the anchor domain, it should be appreciated that the reporter can also be designed to be linked to the 3' end of the anchor domain (making the reporter at the 3' end of the probe and the anchor at the 5' end of the probe).

A "non-nucleoside linker" separates the anchor domain and the reporter domain. A skilled artisan would recognize that any suitable non-nucleoside linker can be used. Routine experiments can be used to determine optimum characteristics for the linker. The anchor domain and the reporter domain are designed to bind to regions in the target nucleic acid adjacent to each other. Therefore, in some embodiments, the anchor domain and the reporter domain can be designed to be relatively close together (e.g., no more than 1, 2, 3, 4, or 5 nucleotides intervening when the reporter and anchor domains both hybridize to the target). The linker will generally be sufficiently flexible to allow the reporter to hybridize, or not, when the anchor is hybridized to an adjacent sequence in the target. The non-nucleoside linker functions to keep the spacing between the anchor domain and the reporter domain. The spacing between the anchor domain and the reporter domain can therefore be adjusted by using a non-nucleotide linker of various lengths.

The non-nucleoside linker can be, for example, aliphatic, aromatic, aryl, cyclic, chiral, achiral, a peptide, a carbohydrate, a lipid, a fatty acid, tri-, tetra-, penta-, hexa-polyethylene glycol (HEG) or poly-polyethylene glycol (PEG), or a heterocyclic moiety. Other conventional non-nucleoside linkers employ homobifunctional and heterobifunctional crosslinking reagents. Homobifunctional reagents carry two identical functional groups, whereas heterobifunctional reagents contain two dissimilar functional groups to link the biologics to the bioadhesive. A vast majority of the heterobifunctional cross-linking agents contain a primary amine-reactive group and a thiol-reactive group. Covalent crosslinking agents are selected from reagents capable of forming disulfide (S--S), glycol (--CH(OH)--CH(OH)--), azo (--N=N--), sulfone (-S(=O2--), ester (--C(=O)--O--), or amide (--C(=O)--N--) bridges.

The Cobra probe is designed to have different combinations of the anchor nucleic acid domain, the reporter nucleic acid domain and the non-nucleoside linker. In some embodiments, the linker can be attached to the terminus of the anchor nucleic acid domain or the reporter nucleic acid domain, e.g. 3' or 5' position of the anchor nucleic acid domain or the reporter nucleic acid domain. Alternatively, the linker can be attached to other, internal positions of one or both nucleic acid domains of the probe, as long as the linkage or attachment does not interfere with the functions of the probe, e.g., hybridization of the probe with the intended target nucleic acid. For example, the linker can be linked to the reporter domain within, e.g., 1, 2, 3, 4, or more nucleotides of the 3' or 5' end of the reporter domain. Similarly, the linker can be linked to the anchor domain within, e.g., 1, 2, 3, 4, or more nucleotides of the 3' or 5' end of the anchor domain. The anchor nucleic acid domain and the reporter nucleic acid domain can be attached to various positions of the non-nucleoside linker. In some embodiments, these nucleic acid domains are attached to the terminus of the linker. Optionally, they can be attached to different positions of the linker.

### Polymerase Chain Reaction (PCR)

U.S. Pat. Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in the present invention include oligonucleotides capable of acting as a point of initiation of nucleic acid synthesis within *tcdC* nucleic acid sequences. A primer can be purified from a restriction digest by conventional methods, or it can be produced synthetically. The primer is preferably single-stranded for maximum efficiency in amplification, but the primer can be double-stranded. Double-stranded primers are first denatured, i.e., treated to separate the strands. One method of denaturing double stranded nucleic acids is by heating.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, e.g., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min).

If the double-stranded template nucleic acid is denatured by heat, the reaction mixture is allowed to cool to a temperature that promotes annealing of each primer to its target sequence on the *tcdC* nucleic acid. The temperature for annealing is usually from about 35°C to about 65°C (e.g., about 40°C to about 60°C; about 45°C to about 50°C). Annealing times can be from about 10 sec to about 1 min (e.g., about 20 sec to about 50 sec; about 30 sec to about 40 sec). The reaction mixture is then adjusted to a temperature at which the activity of the polymerase is promoted or optimized, i.e., a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (e.g., the temperature for extension generally ranges from about 40°C to about 80°C (e.g., about 50°C to about 70°C; about 60°C). Extension times can be from about 10 sec to about 5 min (e.g., about 30 sec to about 4 min; about 1 min to about 3 min; about 1 min 30 sec to about 2 min).

PCR assays can employ *C. difficile* nucleic acid such as RNA or DNA (cDNA). The template nucleic acid need not be purified; it may be a minor fraction of a complex mixture, such as C. *difficile* nucleic acid contained in human cells. *C. difficile* nucleic acids may be extracted from a biological sample by routine techniques such as those described in Diagnostic Molecular Microbiology: Principles and Applications (Persing et al. (eds), 1993, American Society for Microbiology, Washington D.C.). Nucleic acids can be obtained from any number of sources, such as plasmids, or natural sources including bacteria, yeast, viruses, organelles, or higher organisms such as plants or animals.

Oligonucleotide primers are combined with PCR reagents under reaction conditions that induce primer extension. For example, chain extension reactions generally include 50 mM KCl, 10 mM Tris-HCl (pH 8.3), 15 mM MgCl₂, 0.001% (w/v) gelatin, 0.5-1.0 µg denatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target *tcdC* nucleic acid molecule. The limiting factors in the reaction are the amounts of primers, thermostable enzyme, and nucleoside triphosphates present in the reaction. The cycling steps (i.e., denaturation, annealing, and extension) are preferably repeated at least once. For use in detection, the number of cycling steps will depend, e.g., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence Resonance Energy Transfer (FRET)

FRET technology (see, for example, U.S. Pat. Nos. 4,996,143, 5,565,322, 5,849,489, and 6,162,603) is based on a concept that when a donor fluorescent moiety and a corresponding acceptor fluorescent moiety are positioned within a certain distance of each other, energy transfer takes place between the two fluorescent moieties that can be visualized or otherwise detected and/or quantitated. The donor typically transfers the energy to the acceptor when the donor is excited by light radiation with a suitable wavelength. The acceptor typically re-emits the transferred energy in the form of light radiation with a different wavelength.

In one example, a oligonucleotide probe can contain a donor fluorescent moiety and a corresponding quencher, which dissipates the transferred energy in a form other than light. When the probe is intact, energy transfer typically occurs between the two fluorescent moieties such that fluorescent emission from the donor fluorescent moiety is quenched. During an extension step of a polymerase chain reaction, a probe bound to an amplification product is cleaved by the 5' to 3' exonuclease activity of, e.g., a Taq Polymerase such that the fluorescent emission of the donor fluorescent moiety is no longer quenched. Exemplary probes for this purpose are described in, e.g., U.S. Pat. Nos. 5,210,015, 5,994,056, and 6,171,785. Commonly used donor-acceptor pairs include the FAM-TAMRA pair. Commonly used quenchers are DABCYL and TAMRA. Commonly used dark quenchers include BlackHole Quenchers^{™} (BHQ-1, BHQ-2), (Biosearch Technologies, Inc., Novato, Cal.), Iowa Black^{™}, (Integrated DNA Tech., Inc., Coralville, Iowa), BlackBerry^{™} Quencher 650 (BBQ-650), (Berry & Assoc., Dexter, Mich.).

In another example, two oligonucleotide probes, each containing a fluorescent moiety, can hybridize to an amplification product at particular positions determined by the complementarity of the oligonucleotide probes to the *tcdC* target nucleic acid sequence. Upon hybridization of the oligonucleotide probes to the amplification product nucleic acid at the appropriate positions, a FRET signal is generated. Hybridization temperatures can range from about 35° C. to about 65° C. for about 10 sec to about 1 min.

Fluorescent analysis can be carried out using, for example, a photon counting epifluorescent microscope system (containing the appropriate dichroic mirror and filters for monitoring fluorescent emission at the particular range), a photon counting photomultiplier system, or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties "corresponding" refers to an acceptor fluorescent moiety having an emission spectrum that overlaps the excitation spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced there between. Fluorescent donor and corresponding acceptor moieties are generally chosen for (a) high efficiency Forster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm). Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC Red 640, LC Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate, or other chelates of Lanthanide ions (e.g., Europium, or Terbium). Donor and acceptor fluorescent moieties can be obtained, for example, from Molecular Probes (Junction City, Oreg.) or Sigma Chemical Co. (St. Louis, Mo.).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm for the purpose of the present invention is the distance in Angstroms (Å) from the nucleotide base to the fluorescent moiety. In general, a linker arm is from about 10 Å to about 25 Å. The linker arm may be of the kind described in WO 84/03285. WO 84/03285 also discloses methods for attaching linker arms to a particular nucleotide base, and also for attaching fluorescent moieties to a linker arm.

An acceptor fluorescent moiety such as an LC Red 640-NHS-ester can be combined with C6-Phosphoramidites (available from ABI (Foster City, Calif.) or Glen Research (Sterling, Va.)) to produce, for example, LC Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, Mass.)), amide-linkers (fluorescein-NHS-ester-derived, such as fluorescein-CPG from BioGenex (San Ramon, Calif.)), or 3'-amino-CPGs that require coupling of a fluorescein-NHS-ester after oligonucleotide synthesis.

### Detection of Clostridium difficile

The present invention provides methods for detecting the presence or absence of the single-base deletion at nucleotide position 117 in the *tcdC* gene (tcdC Δ117) of C. *difficile* in a biological or non-biological sample. The claimed methods can avoid problems of sample contamination, e.g., carry-over contamination from run to run, false negatives, e.g., sensitivity, and false positives, e.g., specificity. The methods include performing at least one cycling step that includes amplifying a portion of a *tcdC* nucleic acid molecule from a sample using a pair of *tcdC* primers, and a FRET detecting step. Multiple cycling steps are performed, preferably in a thermocycler. Methods of the invention can be performed using the *tcdC* primers and Cobra probes to detect the presence of tcdC Δ117 and the detection of tcdC Δ117 indicates the presence of an epidemic ribotype of C. *difficile* in the sample.

As described herein, amplification products can be detected using labeled hybridization probes that take advantage of FRET technology. One FRET format utilizes TaqMan^{®} technology to detect the presence or absence of an amplification product, and hence, the presence or absence of C. *difficile.* TaqMan^{®} technology utilizes one single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hybridization probe binds to the target DNA (i.e., the amplification product) and is degraded by the 5' to 3' exonuclease activity of the Taq Polymerase during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM^{®} 7700 Sequence Detection System (Applied Biosystems, Foster City, CA) uses TaqMan^{®} technology, and is suitable for performing the methods described herein for detecting the presence or absence of C. *difficile* in the sample.

Molecular beacons in conjunction with FRET can also be used to detect the presence of an amplification product using the real-time PCR methods of the invention. Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (e.g., a hairpin). As a result of secondary structure formation within the probe, both fluorescent moieties are in spatial proximity when the probe is in solution. After hybridization to the target nucleic acids (i.e., amplification products), the secondary structure of the probe is disrupted and the fluorescent moieties become separated from one another such that after excitation with light of a suitable wavelength, the emission of the first fluorescent moiety can be detected.

Another common format of FRET technology utilizes two hybridization probes. Each probe can be labeled with a different fluorescent moiety and are generally designed to hybridize in close proximity to each other in a target DNA molecule (e.g., an amplification product). A donor fluorescent moiety, for example, fluorescein, is excited at 470 nm by the light source of the LightCycler^{®} Instrument. During FRET, the fluorescein transfers its energy to an acceptor fluorescent moiety such as LightCycler^{®}-Red 640 (LC Red 640) or LightCycler^{®}-Red 705 (LC Red 705). The acceptor fluorescent moiety then emits light of a longer wavelength, which is detected by the optical detection system of the LightCycler^{®} instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target DNA molecules (e.g., the number of C. *difficile* genomes). If amplification of *tcdC* nucleic acid occurs and an amplification product is produced, the step of hybridizing results in a detectable signal based upon FRET between the members of the pair of probes.

Generally, the presence of FRET indicates the presence of *C. difficile* in the sample, and the absence of FRET indicates the absence of *C. difficile* in the sample. Inadequate specimen collection, transportation delays, inappropriate transportation conditions, or use of certain collection swabs (calcium alginate or aluminum shaft) are all conditions that can affect the success and/or accuracy of a test result, however. Using the methods disclosed herein, detection of FRET within, e.g., 45 cycling steps is indicative of a *C. difficile* infection.

Representative biological samples that can be used in practicing the methods of the invention include, but are not limited to dermal swabs, nasal swabs, wound swabs, blood cultures, skin, and soft tissue infections. Collection and storage methods of biological samples are known to those of skill in the art. Biological samples can be processed (e.g., by nucleic acid extraction methods and/or kits known in the art) to release *C. difficile* nucleic acid or in some cases, the biological sample can be contacted directly with the PCR reaction components and the appropriate oligonucleotides.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the *tcdC* probes from the *tcdC* amplification product can confirm the presence or absence of C. *difficile* in the sample.

Within each thermocycler run, control samples are cycled as well. Positive control samples can amplify *C. difficile* nucleic acid control template (other than *tcdC*) using, for example, control primers and control probes. Positive control samples can also amplify, for example, a plasmid construct containing *tcdC* nucleic acid molecules. Such a plasmid control can be amplified internally (e.g., within the sample) or in a separate sample run side-by-side with the patients' samples. Each thermocycler run can also include a negative control that, for example, lacks *C. difficile* template DNA. Such controls are indicators of the success or failure of the amplification, hybridization, and/or FRET reaction. Therefore, control reactions can readily determine, for example, the ability of primers to anneal with sequence-specificity and to initiate elongation, as well as the ability of probes to hybridize with sequence-specificity and for FRET to occur.

In an embodiment, the methods of the invention include steps to avoid contamination. For example, an enzymatic method utilizing uracil-DNA glycosylase is described in U.S. Pat. Nos. 5,035,996, 5,683,896 and 5,945,313 to reduce or eliminate contamination between one thermocycler run and the next.

Conventional PCR methods in conjunction with FRET technology can be used to practice the methods of the invention. In one embodiment, a LightCycler^{®} instrument is used. The following patent applications describe real-time PCR as used in the LightCycler^{®} technology: WO 97/46707, WO 97/46714, and WO 97/46712.

The LightCycler^{®} can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (e.g., SYBR^{®} Green or SYBR^{®} Gold (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

It is understood that the present invention is not limited by the configuration of one or more commercially available instruments.

### Articles of Manufacture/Kits

Also provided are articles of manufacture or kits to detect the single-base deletion at nucleotide position 117 in the *tcdC* gene (tcdC Δ117) of *C. difficile.* An article of manufacture according to the present invention can include primers and probes used to detect tcdC Δ117, together with suitable packaging materials. Representative primers and probes for detection of tcdC Δ117 are capable of hybridizing to *tcdC* nucleic acid molecules. In addition, the kits may also include suitably packaged reagents and materials needed for DNA immobilization, hybridization, and detection, such solid supports, buffers, enzymes, and DNA standards. Methods of designing primers and probes are disclosed herein, and representative examples of primers and probes that amplify and hybridize to *tcdC* nucleic acid molecules are provided.

Articles of manufacture can also include one or more fluorescent moieties for labeling the probes or, alternatively, the probes supplied with the kit can be labeled. For example, an article of manufacture may include a donor fluorescent moiety for labeling one of the *tcdC* probes and an acceptor fluorescent moiety for labeling the other *tcdC* probe, respectively. Examples of suitable FRET donor fluorescent moieties and corresponding acceptor fluorescent moieties are provided above.

Articles of manufacture can also contain a package insert or package label having instructions thereon for using the *tcdC* primers and probes to detect tcdC Δ117 in a sample. Articles of manufacture may additionally include reagents for carrying out the methods disclosed herein (e.g., buffers, polymerase enzymes, co-factors, or agents to prevent contamination). Such reagents may be specific for one of the commercially available instruments described herein.

The invention will be further described in the following examples, which do not limit the scope of the invention described in the claims.

### EXAMPLES

The following examples and figures are provided to aid the understanding of the present invention, the true scope of which is set forth in the appended claims.

### EXAMPLE 1: Design of Cobra Probes For Detection of tcdC Δ117

To detect the single nucleotide deletion at nucleotide position 117 in *tcdC* (tcdC Δ117), traditional probes and Cobra probes were designed for the mutant sequence representing the single nucleotide deletion. JN 944619is the NCBI reference sequence for the *C. difficile* strain CD1395 used in designing primers and probes for detecting the tcdC Δ117. Genomic and Plasmid DNA samples were tested at various copy levels and amplification was performed using real-time PCR on the **cobas z** 480 instrument (Roche Molecular Systems, Inc).

The nucleotide sequences of the Cobra probes and the "regular" sense probe that are perfectly matched to the Δ117 single-nucleotide deletion in the *tcdC* gene and the sequences of forward and reverse primers are listed on Table 1.

**TABLE 1:**

| **NAME** | **SEQUENCE** | **MODIFICATIONS** | **SEQ ID NO:** |
|---|---|---|---|
| Δ117-Cobra FQ9 | 5'- FJJJGGTGJGQEEGGCAATATAT CCTCACCAGCTTGTTCTGAAP -3' | F= FAM; J = propynyl-dU; Q= BHQ-2; E= HEG linker; P= phosphate | 1 |
| Δ117-Cobra FQ6 | 5'- FJJJGGTQGJGEEGGCAATATAT CCTCACCAGCTTGTTCTGAAP -3' | F= FAM; J = propynyl-dU; Q= BHQ-2; E= HEG linker; P= phosphate | 2 |
| Δ117-sense FQ4 | 5'- FTGGTQGTGTTTTTTGGCP -3' | F= FAM;; Q= BHQ-2; P= phosphate | 3 |
| KMTCDC 48F | 5’- AGGAAGCTCTAAGAAAATAA TTAAATTC -3’ | | 4 |
| KMTCDC 155R | 5'- GAACAAGCTGGTGAGGAT -3' | | 5 |
| KMTCDC 48FPDU | 5'- AGGAAGCTCTAAGAAAATA ATTAAATJ -3' | J= propynyl-dU | 6 |
| KMTCDC 155RPDU | 5'- AACAAGCTGGTGAGGAJ -3' | J = propynyl-dU | 7 |

### EXAMPLE 2: Real-Time PCR Assay Conditions

**TABLE 2 PCR Amplification Reagents**

| ***Master Mix Component*** | ***Final Conc (50uL)*** | |
|---|---|---|
| DMSO | 5.4 | % |
| NaN3 | 0.030 | % |
| Potassium acetate | 120.0 | mM |
| Glycerol | 3.0 | % |
| Tween 20 | 0.02 | % |
| EDTA | 43.9 | uM |
| Tricine | 60.0 | mM |
| Aptamer | 0.18 | uM |
| UNG Enzyme | 5.0 | U |
| Z05-SP-PZ Polymerase | 30.0 | U |
| dATP | 521.70 | uM |
| dCTP | 521.70 | uM |
| dGTP | 521.70 | uM |
| dUTP | 1043.40 | uM |
| Forward primer oligonucleotides-target | 0.10-0.30 | µM |
| Reverse primer oligonucleotides-target | 0.15-0.30 | µM |
| Probe oligonucleotides-target | 0.10 | µM |
| Manganese Acetate | 3.80 | mM |

The following table shows the typical thermoprofile used for PCR amplification reaction

**TABLE 3 PCR Thermoprofile**

| **Step** | **Target (°C)** | **Hold time (mm:ss)** | **Ramp Rate (°C / s)** | **Cycles** |
|---|---|---|---|---|
| Pre-PCR | 50 | 02:00 | 4.4 | 1 |
| | 94 | 00:05 | 4.4 | 1 |
| 1. Meas | 95 | 00:05 | 4.4 | 5 |
| | 55 | 00:30 | 2.2 | 5 |
| 2. Meas. | 91 | 00:05 | 4.4 | 45 |
| | 58 | 00:25 | 2.2 | 45 |
| Post-PCR | 40 | 02:00 | 2.2 | 1 |

### EXAMPLE 3: Results of PCR Assay For Detection of tcdC Δ117

Genomic DNA from three strains of *C. difficile* were used as templates. Template from Strain 12840 represents the wild type *C. difficile.* Template from strain 11313 represents a mutant *C. difficile* with an SNP at nucleotide position 120 of the *tcdC* gene that produces a binary toxin but is wild-type for Δ117. Template from strain 11314 represents the mutant C. difficile strain with the tcdC Δ117 gene. Growth curves from a PCR experiment using the regular sense probe Δ117-sense FQ4 (SEQ ID NO:3) with forward primer (SEQ ID NO:4) and reverse primer (SEQ ID NO:5) are shown in FIG. 3. While the highest signals were observed with the 11314 template (tcdC Δ117) at 100 (1e2) and 1000 (1e3) copies/reaction concentration, no signal was observed with the 11314 template at 10 (1e1) copies/reaction concentration. Also, discernible signals could also be detected from the strain11313 template (SNP 120) at 10⁵ (1e5) and 10⁶ (1e6) copies/reaction and also from the 12840 wild-type template at 10⁵ copies/reaction concentrations. In contrast, when the cobra probes Δ117-Cobra FQ9 (SEQ ID NO: 1) or Δ117-Cobra FQ6 (SEQ ID NO:2) were used with the same forward and reverse primers, not only was there higher sensitivity for the 11314 tcdC Δ117 template (with signals detected at 10 [1e1] copies/reaction) but also, reduced or no signals were seen for the strain 11313 and strain 12840 templates at either 10⁵ (1e5) or 10⁶ (1e6) copies/reaction concentrations (FIG. 4 and FIG. 5).

While the foregoing invention has been described in some detail for purposes of clarity and understanding, it will be clear to one skilled in the art from a reading of this disclosure that various changes in form and detail can be made. For example, all the techniques and apparatus described above can be used in various combinations.

## Claims

1. A method of detecting a single-base deletion at nucleotide position 117 in the *tcdC* gene of Ribotype 027 strains of *C. difficile* (tcdC Δ117) in a sample, the method comprising:
- performing an amplifying step comprising contacting the sample with a set of *tcdC* primers to produce an amplification product if a *tcdC* nucleic acid is present in the sample;
- performing a hybridizing step comprising contacting the amplification product with a detectable tcdC Δ117 probe; and
- detecting the presence or absence of a signal from said detectable tcdC Δ117 probe, wherein the presence of the signal is indicative of the presence of tcdC Δ117 in the sample and wherein the absence of the signal is indicative of the absence of tcdC Δ117 in the sample;
wherein said detectable tcdC Δ117 probe is a Cobra probe that comprises a reporter domain having a contiguous sequence that is fully complementary to the single-base deletion at nucleotide position 117 and wherein said reporter domain is separated from an anchor domain by a non-nucleoside linker comprising one or more hexaethylene glycol (HEG); and wherein said Cobra probe comprises a sequence selected from the group consisting of SEQ ID NOs: 1 and 2, or a complement thereof.

2. The method of claim 1, wherein:
- the hybridizing step comprises contacting the amplification product with said Cobra probe that is labeled with a donor fluorescent moiety and a corresponding acceptor fluorescent moiety; and
- the detecting step comprises detecting the presence or absence of fluorescence resonance energy transfer (FRET) between the donor fluorescent moiety and the acceptor fluorescent moiety of the Cobra probe, wherein the presence or absence of fluorescence is indicative of the presence or absence of tcdC Δ117 in the sample.

3. The method of any one of claims 1 to 2, wherein said amplification employs a polymerase enzyme having 5' to 3' exonuclease activity.

4. The method of any one of claims 1 to 3, wherein the tcdC Δ117 probe comprises a nucleic acid sequence that permits secondary structure formation, wherein the secondary structure formation results in spatial proximity between the donor fluorescent moiety and the acceptor fluorescent moiety.

5. The method of any one of claims 2 to 4, wherein said second fluorescent moiety is a quencher.

6. A kit for detecting a single-base deletion at nucleotide position 117 in the *tcdC* gene of Ribotype 027 strains *of C. difficile* (tcdC Δ117) comprising:
- one pair of oligonucleotide primers, each oligonucleotide primer capable of hybridizing to opposite strands of a subsequence of the *tcdC* gene containing nucleotide position 117;
- a detectably labeled oligonucleotide probe comprising a sequence selected from the group consisting of SEQ ID NOs: 1 and 2, or a complement thereof.

7. The kit of claim 6, wherein the detectably labeled oligonucleotide comprises a donor fluorescent moiety and a corresponding acceptor fluorescent moiety.

8. The kit of claim 7, wherein the acceptor fluorescent moiety is a quencher.

9. The kit of any one of claims 6 to 8, further comprising nucleoside triphosphates, a nucleic acid polymerase, and buffers necessary for the function of the nucleic acid polymerase.

10. An oligonucleotide comprising a sequence of oligonucleotides selected from the group consisting of SEQ ID NOs: 1 and 2, or a complement thereof.

11. The oligonucleotide of claim 10, wherein the oligonucleotide comprises at least one modified nucleotide.

12. The oligonucleotide of any one of claims 10 to 11, wherein the oligonucleotide comprises at least one conservatively modified variation.

13. The oligonucleotide of any one of claims 10 to 12, further comprising one or more detectable label.

14. The oligonucleotide of claim 13, wherein the oligonucleotide comprises at least one labeling moiety and/or at least one quencher moiety.

## Patentansprüche

1. Verfahren zum Nachweisen einer Einzelbasendeletion an Nukleotidposition 117 im *tcdC-*Gen von Ribotyp-027-Stämmen von *C. difficile* (tcdC Δ117) in einer Probe, wobei das Verfahren Folgendes umfasst:
- Durchführen eines Amplifizierungsschrittes, umfassend das Inkontaktbringen der Probe mit einem Satz von *tcdC*-Primern, um ein Amplifikationsprodukt zu produzieren, wenn eine *tcdC*-Nukleinsäure in der Probe vorliegt;
- Durchführen eines Hybridisierungsschrittes, umfassend das Inkontaktbringen des Amplifikationsproduktes mit einer nachweisbaren tcdC Δ117-Sonde; und
- Nachweisen des Vorliegens oder Nichtvorliegens eines Signals aus der nachweisbaren tcdC Δ117-Sonde, wobei das Vorliegen des Signals das Vorliegen von tcdC Δ117 in der Probe angibt und wobei das Nichtvorliegen des Signals das Nichtvorliegen von tcdC Δ117 in der Probe angibt;
wobei die nachweisbare tcdC Δ117-Sonde eine Cobra-Sonde ist, die eine Reporterdomäne mit einer zusammenhängenden Sequenz umfasst, die vollständig komplementär ist zu der Einzelbasendeletion an Nukleotidposition 117, und wobei die Reporterdomäne von einer Ankerdomäne durch einen Nicht-Nukleosid-Linker getrennt wird, der ein oder mehrere Hexaethylenglycol(e) (HEG) umfasst; und wobei die Cobra-Sonde eine Sequenz, ausgewählt aus der Gruppe, bestehend aus SEQ ID NO:1 und 2, oder ein Komplement davon umfasst.

2. Verfahren nach Anspruch 1, wobei:
- der Hybridisierungsschritt das Inkontaktbringen des Amplifikationsproduktes mit der Cobra-Sonde umfasst, die mit einer Donor-Fluoreszenzeinheit und einer entsprechenden Akzeptor-Fluoreszenzeinheit markiert ist; und
- der Nachweisschritt das Nachweisen des Vorliegens oder Nichtvorliegens von Fluoreszenz-Resonanz-Energietransfer (FRET) zwischen der Donor-Fluoreszenzeinheit und der Akzeptor-Fluoreszenzeinheit der Cobra-Sonde umfasst, wobei das Vorliegen oder Nichtvorliegen von Fluoreszenz das Vorliegen oder Nichtvorliegen von tcdC Δ117 in der Probe angibt.

3. Verfahren nach einem der Ansprüche 1 bis 2, wobei die Amplifikation ein Polymeraseenzym mit 5'- zu 3'-Exonukleaseaktivität einsetzt.

4. Verfahren nach einem der Ansprüche 1 bis 3, wobei die tcdC Δ117-Sonde eine Nukleinsäuresequenz umfasst, die die Bildung einer Sekundärstruktur ermöglicht, wobei die Bildung einer Sekundärstruktur zu räumlicher Nähe zwischen der Donor-Fluoreszenzeinheit und der Akzeptor-Fluoreszenzeinheit führt.

5. Verfahren nach einem der Ansprüche 2 bis 4, wobei die zweite Fluoreszenzeinheit ein Quencher ist.

6. Kit zum Nachweisen einer Einzelbasendeletion an Nukleotidposition 117 in dem *tcdC-*Gen von Ribotyp-027-Stämmen von *C. difficile* (tcdC Δ117), umfassend:
- ein Paar von Oligonukleotid-Primern, wobei jeder Oligonukleotid-Primer an gegenüberliegende Stränge einer Teilsequenz des *tcdC-Gens,* die Nukleotidposition 117 enthält, hybridisieren kann;
- eine nachweisbar markierte Oligonukleotidsonde, die eine Sequenz, ausgewählt aus der Gruppe, bestehend aus SEQ ID NO:1 und 2, oder ein Komplement davon umfasst.

7. Kit nach Anspruch 6, wobei das nachweisbar markierte Oligonukleotid eine Donor-Fluoreszenzeinheit und eine entsprechende Akzeptor-Fluoreszenzeinheit umfasst.

8. Kit nach Anspruch 7, wobei die Akzeptor-Fluoreszenzeinheit ein Quencher ist.

9. Kit nach einem der Ansprüche 6 bis 8, ferner umfassend Nukleosidtriphosphate, eine Nukleinsäurepolymerase und Puffer, die für die Funktion der Nukleinsäurepolymerase notwendig sind.

10. Oligonukleotid, das eine Sequenz von Oligonukleotiden, ausgewählt aus der Gruppe, bestehend aus SEQ ID NO:1 und 2, oder ein Komplement davon umfasst.

11. Oligonukleotid nach Anspruch 10, wobei das Oligonukleotid mindestens ein modifiziertes Nukleotid umfasst.

12. Oligonukleotid nach einem der Ansprüche 10 bis 11, wobei das Oligonukleotid mindestens eine konservativ modifizierte Variation umfasst.

13. Oligonukleotid nach einem der Ansprüche 10 bis 12, ferner umfassend eine oder mehrere nachweisbare Markierung(en).

14. Oligonukleotid nach Anspruch 13, wobei das Oligonukleotid mindestens eine Markierungseinheit und/oder mindestens eine Quencher-Einheit umfasst.

## Revendications

1. Procédé de détection d'une délétion de base unique en position nucléotidique 117 dans le gène *tcdC* de souches du ribotype 027 de *C. difficile* (tcdC Δ117) dans un échantillon, le procédé comprenant :
- la réalisation d'une étape d'amplification comprenant la mise en contact de l'échantillon avec un ensemble d'amorces de *tcdC* pour produire un produit d'amplification si un acide nucléique de *tcdC* est présent dans l'échantillon ;
- la réalisation d'une étape d'hybridation comprenant la mise en contact du produit d'amplification avec une sonde de tcdC Δ117 détectable ; et
- la détection de la présence ou de l'absence d'un signal de ladite sonde de tcdC Δ117 détectable, dans laquelle la présence du signal indique la présence de tcdC Δ117 dans l'échantillon et dans laquelle l'absence du signal indique l'absence de tcdC Δ117 dans l'échantillon ;
dans lequel ladite sonde de tcdC Δ117 détectable est une sonde Cobra qui comprend un domaine rapporteur ayant une séquence contiguë qui est totalement complémentaire de la délétion de base unique en position nucléotidique 117 et dans lequel ledit domaine rapporteur est séparé d'un domaine d'ancrage par un lieur non nucléosidique comprenant un ou plusieurs hexaéthylène glycols (HEG) ; et dans lequel ladite sonde Cobra comprend une séquence choisie dans le groupe constitué par les SEQ ID NO : 1 et 2, ou un complément de celle-ci.

2. Procédé selon la revendication 1, dans lequel :
- l'étape d'hybridation comprend la mise en contact du produit d'amplification avec ladite sonde Cobra qui est marquée avec une fraction fluorescente donneuse et une fraction fluorescente acceptrice correspondante ; et
- l'étape de détection comprend la détection de la présence ou de l'absence d'un transfert d'énergie par résonance de fluorescence (FRET) entre la fraction fluorescente donneuse et la fraction fluorescente acceptrice de la sonde Cobra, dans laquelle la présence ou l'absence de fluorescence indique la présence ou l'absence de tcdC Δ117 dans l'échantillon.

3. Procédé selon l'une quelconque des revendications 1 à 2, dans lequel ladite amplification utilise une enzyme polymérase ayant une activité exonucléase 5' vers 3'.

4. Procédé selon l'une quelconque des revendications 1 à 3, dans lequel la sonde de tcdC Δ117 comprend une séquence d'acide nucléique qui permet une formation de structure secondaire, dans lequel la formation de structure secondaire conduit à une proximité spatiale entre la fraction fluorescente donneuse et la fraction fluorescente acceptrice.

5. Procédé selon l'une quelconque des revendications 2 à 4, dans lequel ladite seconde fraction fluorescente est un extincteur.

6. Kit pour la détection d'une délétion de base unique en position nucléotidique 117 dans le gène *tcdC* de souches du ribotype 027 de *C. difficile* (tcdC Δ117) comprenant :
- une paire d'amorces oligonucléotidiques, chaque amorce oligonucléotidique étant capable de s'hybrider à des brins opposés d'une sous-séquence du gène *tcdC* contenant le position nucléotidique 117 ;
- une sonde oligonucléotidique marquée de manière détectable comprenant une séquence choisie dans le groupe constitué par les SEQ ID NO : 1 et 2, ou un complément de celle-ci.

7. Kit selon la revendication 6, dans lequel l'oligonucléotide marqué de manière détectable comprend une fraction fluorescente donneuse et une fraction fluorescente acceptrice correspondante.

8. Kit selon la revendication 7, dans lequel la fraction fluorescente acceptrice est un extincteur.

9. Kit selon l'une quelconque des revendications 6 à 8, comprenant en outre des triphosphates de nucléoside, une polymérase d'acide nucléique et des tampons nécessaires pour la fonction de la polymérase d'acide nucléique.

10. Oligonucléotide comprenant une séquence d'oligonucléotides choisie dans le groupe constitué par les SEQ ID NO : 1 et 2, ou un complément de celle-ci.

11. Oligonucléotide selon la revendication 10, dans lequel l'oligonucléotide comprend au moins un nucléotide modifié.

12. Oligonucléotide selon l'une quelconque des revendications 10 à 11, dans lequel l'oligonucléotide comprend au moins une variation modifiée de manière conservatrice.

13. Oligonucléotide selon l'une quelconque des revendications 10 à 12, comprenant en outre un ou plusieurs marqueurs détectables.

14. Oligonucléotide selon la revendication 13, dans lequel l'oligonucléotide comprend au moins une fraction de marquage et/ou au moins une fraction extinctrice.


### SEQUENCE LISTING

<110> Roche Diagnostics GmbH F. Hoffmann-La Roche AG Roche Molecular Systems, Inc.
<120> COBRA PROBES TO DETECT A MARKER FOR EPIDEMIC RIBOTYPES OF CLOSTRIDIUM DIFFICILE
<130> P34018-WO-HS
<150> 62/438,331
   <151> 2016-12-22
<160> 7
<170> PatentIn version 3.5
<210> 1
   <211> 39
   <212> DNA
   <213> Artificial Sequence
<220>
   <223> Delta 117- Cobra FQ9 probe
<220>
   <221> misc_feature
   <223> 5' FAM
<220>
   <221> misc_feature
   <223> 3' phosphate
<220>
   <221> modified_base
   <222> (1)..(3)
   <223> propynyl-dU
<220>
   <221> modified_base
   <222> (8)..(8)
   <223> propynyl-dU
<220>
   <221> misc_feature
   <222> (9)..(10)
   <223> BHQ-2 quencher
<220>
   <221> misc_feature
   <222> (9)..(10)
   <223> HEG linker X2
<400> 1
   tttggtgtgg gcaatatatc ctcaccagct tgttctgaa 39
<210> 2
   <211> 39
   <212> DNA
   <213> Artificial Sequence
<220>
   <223> Delta 117- Cobra FQ6 probe
<220>
   <221> misc_feature
   <223> 5' FAM
<220>
   <221> misc_feature
   <223> 3' phosphate
<220>
   <221> modified_base
   <222> (1)..(3)
   <223> propynyl-dU
<220>
   <221> misc_feature
   <222> (6)..(7)
   <223> BHQ-2 quencher
<220>
   <221> modified_base
   <222> (8)..(8)
   <223> propynyl-dU
<220>
   <221> misc_feature
   <222> (9)..(10)
   <223> HEG linker X2
<400> 2
   tttggtgtgg gcaatatatc ctcaccagct tgttctgaa 39
<210> 3
   <211> 16
   <212> DNA
   <213> Artificial Sequence
<220>
   <223> Delta 117- Sense FQ4 probe
<220>
   <221> misc_feature
   <223> 5' FAM
<220>
   <221> misc_feature
   <223> 3' phosphate
<220>
   <221> misc_feature
   <222> (4)..(5)
   <223> BHQ-2 quencher
<400> 3
   tggtgtgttt tttggc 16
<210> 4
   <211> 28
   <212> DNA
   <213> Artificial Sequence
<220>
   <223> Forward primer
<400> 4
   aggaagctct aagaaaataa ttaaattc 28
<210> 5
   <211> 18
   <212> DNA
   <213> Artificial Sequence
<220>
   <223> Reverse primer
<400> 5
   gaacaagctg gtgaggat 18
<210> 6
   <211> 27
   <212> DNA
   <213> Artificial Sequence
<220>
   <223> Forward primer
<220>
   <221> modified_base
   <222> (27)..(27)
   <223> propynyl-dU
<400> 6
   aggaagctct aagaaaataa ttaaatt 27
<210> 7
   <211> 17
   <212> DNA
   <213> Artificial Sequence
<220>
   <223> Reverse primer
<220>
   <221> modified_base
   <222> (17)..(17)
   <223> propynyl-dU
<400> 7
   aacaagctgg tgaggat 17